# EUROPEAN PATENT APPLICATION

(11) **EP 4 681 746 A1**
(43) Date of publication of application: **21.01.2026**
(21) Application number: 25190263.1
(22) Date of filing: 17.07.2025
(51) Int. Cl.: A61M 1/06

(54) **A MILK COLLECTION DEVICE**

(30) Priority: 19.07.2024 GB 202410645
(71) Applicant: Willow Blossom Holdco Limited, London, Greater London EC2V 6DN (GB)
(72) Inventor: CHITTY, Emily, London (GB); SHERIFF, Sophie, London (GB); SCOTT-COLLINS, Callum, London (GB); McGLACKEN, Conor, London (GB); HOWELL, Robin, London (GB); HARRIS, Roy, London (GB)
(74) Representative: Williams Powell

(57) **Abstract**

An in-bra wearable milk collection device for use in a breast pump system. The milk collection device comprises a breast shield, a shell, and a diaphragm. The breast shield includes a nipple tunnel and breast flange. The diaphragm has a dry side for connection to an air pump and a wet side configured to contact milk. The breast shield is removably attachable to the shell and, when attached, the breast shield and shell form a milk container. The breast shield is made from a deformable material such that the breast shield is configured to deform to attach to the shell.

## Description

### FIELD OF THE INVENTION

The present invention relates to a milk collection device for use in a breast pump system. The present invention also relates to a breast pump system.

### BACKGROUND

A breast pump system is a mechanical or electro-mechanical device that extracts milk from the breasts of a lactating woman. A negative pressure, also referred to as a vacuum, is used to simulate suction generated by a feeding child.

Portable breast pumps comprising a milk collection device connected to an external air pump via an air-line are known in the art. The air pump can be battery powered to allow pumping on-the-go without any tethers to electrical sockets or collection stations. The internal breast shield is convex to fit over a breast.

It is desirable to ensure no milk reaches the air-lines. Milk present in the air-lines can interfere with the effectiveness of the vacuum transferred from the air pump. Cleaning the air-lines can be difficult and require specialist cleaning equipment which is not readily available to users at home. Therefore, if milk enters the air-lines it tends to build up in the air-lines over time. There is a need for a milk collection device which prevents milk from reaching the air-lines.

Known milk collection devices comprise breast shields to interface with the breast. In some devices, the user is required to orient the breast shield in a particular direction relative to other components in order for the device to work. This may cause problems if the user incorrectly aligns the breast shield. It also might take the user an extended period of time to correctly align the breast shield. There is a need for a milk collection device which provides fast and easy attachment of the breast shield.

It is desirable to ensure milk collections devices do not slip off the user's breast. Slipping of the breast shield wastes the user's time as they need to reposition the device. It may also cause the user to press the device to their breast throughout use, and/or to negatively impact the suction provided by the breast pump on the user's nipple. There is a need for a milk collection device which provides retention and stability on the breast.

Known milk collections devices have surface features which interfere with the substantially breast shaped profile. For example, connections to air-lines or recesses for alignment purposes. These features inhibit discretion as the pump can be seen through the bra. This defeats the purpose of a discreet, on-the-go breast pump. There is a need for a milk collection device which provides pumping discretion in-bra.

### SUMMARY

There is provided a milk collection device for use in a breast pump system as defined in the appended claims. There is also provided a breast pump system.

### BRIEF DESCRIPTION OF THE DRAWINGS

Specific embodiments are described below by way of example only and with reference to the accompanying drawings in which:
Figure 1 shows a milk collection device according to an embodiment of the invention.
Figures 2A and 2B show a breast shield according to an embodiment of the invention.
Figures 3A and 3B show a shell according to an embodiment of the invention.
Figure 4A shows a diaphragm according to an embodiment of the invention.
Figure 4B shows a milk collection device according to an embodiment of the invention.
Figure 5 shows a cap according to an embodiment of the invention.
Figure 6 shows a non-return valve according to an embodiment of the invention.
Figure 7 shows a milk collection device according to an embodiment of the invention.
Figure 8 shows a milk collection device according to an embodiment of the invention.
Figure 9 shows a milk collection device according to an embodiment of the invention.
Figure 10 shows a milk collection device according to an embodiment of the invention.
Figure 11A shows a shell according to an embodiment of the invention.
Figure 11B shows a breast shield according to an embodiment of the invention.
Figure 12 shows a milk collection device according to an embodiment of the invention.
Figures 13A and 13B show a breast shield according to an embodiment of the invention.
Figure 14 shows an exploded view of a milk collection device according to an embodiment of the invention.

Aspects and features of the present invention are set out in the accompanying claims.

### DETAILED DESCRIPTION OF THE EMBODIMENTS

The terms "breast pump" and "breast pump system" are interchangeable herein.

The terms "milk collection device" and "the device" are interchangeable herein.

The term "dry side" may be used in various contexts herein. For example, the dry side of the diaphragm, the dry side of the milk collection device and the dry side of the breast pump system. The term dry side refers to the parts which do not come into contact with milk during normal use. Similarly, the term "wet side" may be used in various contexts. For example, the wet side of the diaphragm, the wet side of the milk collection device and the wet side of the breast pump system. The term wet side refers to the parts which do come into contact with milk during normal use.

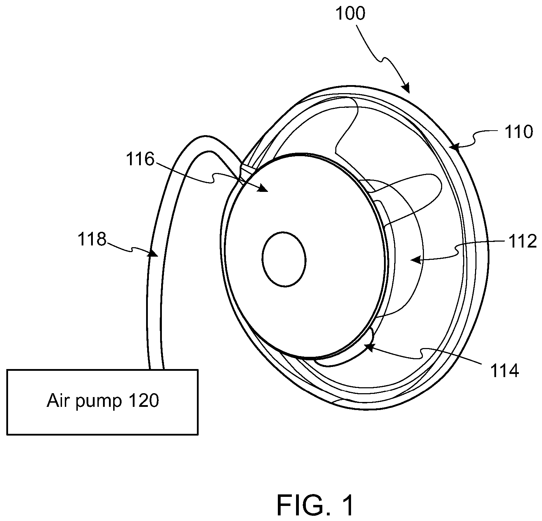

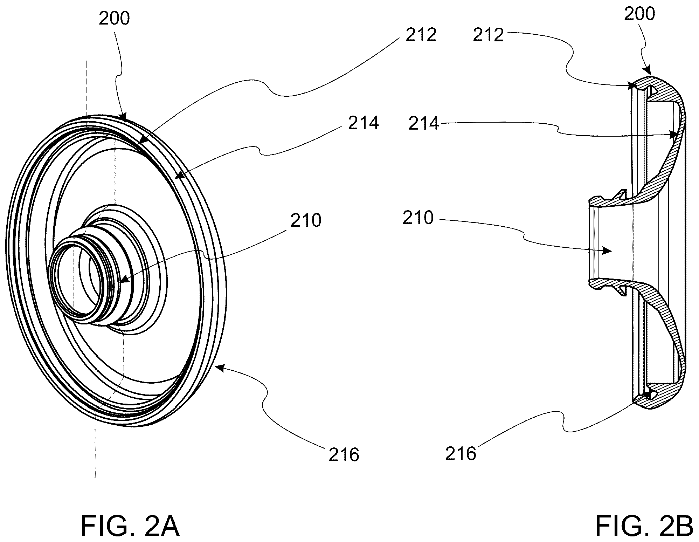

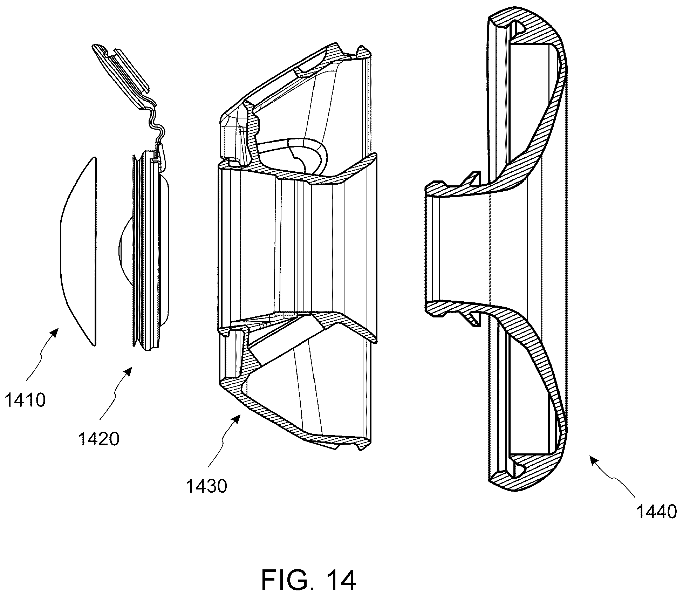

Figure 1 shows an assembled, ready for use, milk collection device 100 according to an embodiment of the invention. Figures 2A to 6 show possible individual component parts of the milk collection device 100. That is, the components shown in Figures 2A to 6 may attach to each other to form the milk collection device 100. Figure 14 shows an exploded view of the possible components in a milk collection device.

The milk collection device 100 may be in-bra wearable such that it is able to be held at least partially within a bra. This is such that the device can be supported by the bra and does not need to be held in the user's hand. The milk collection device 100 when assembled may form a breast-like curved shape such that the device is discreet when held within a bra.

The milk collection device 100 is suitable for use in a breast pump system. The breast pump system may comprise one or more milk collection devices 100, an air pump 120 and/or one or more air-lines 118. The function of the air pump 120 is to create a vacuum, the vacuum used to create suction on the user's breast. The skilled person will understand that in this context the term vacuum relates to a relative negative pressure rather than an absolute vacuum. The one or more air-lines 118 connect the air pump 120 to the milk collection device. The breast pump system may comprise other components, for example: a screen, buttons, a control system, a processor, a memory, valves, solenoids or sensors. A single air pump 120 may connect via two air-lines 118 to two milk collection devices 110 respectively.

Using an alternative but equally valid definition, any milk collection device described herein can be considered to include one or more air-lines.

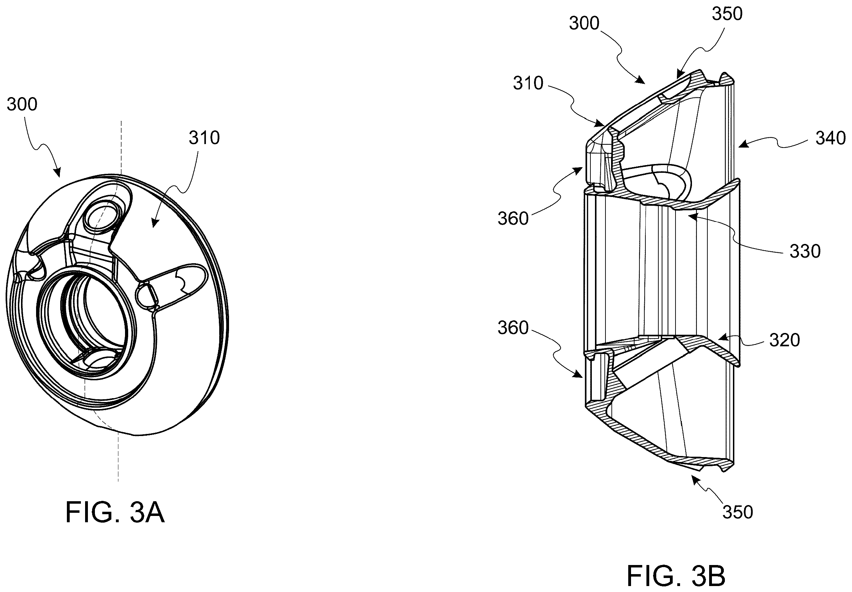

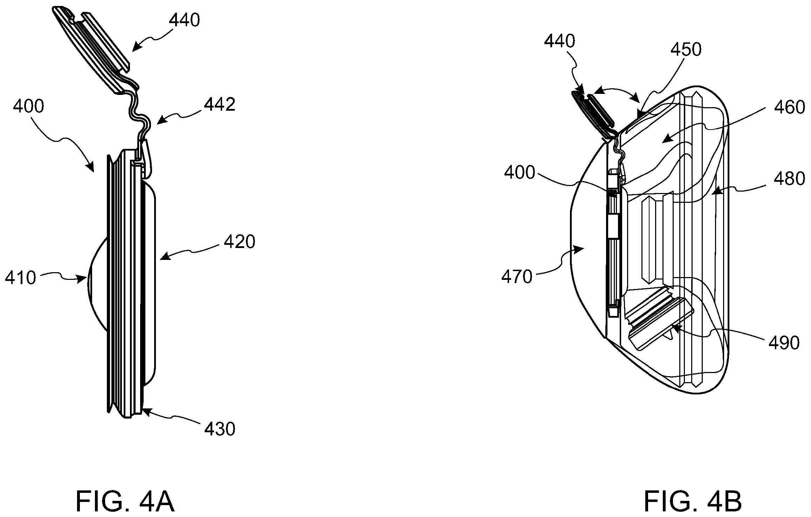

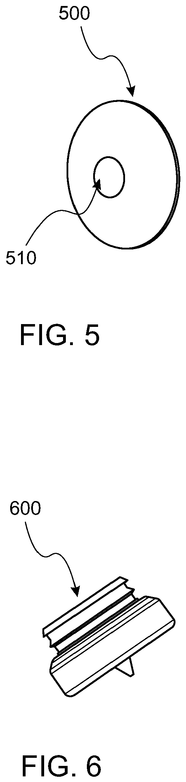

The milk collection device 100 comprises a breast shield 110, an example is depicted in Figures 2A and 2B. The milk collection device 100 comprises a shell 112, an example is depicted in Figures 3A and 3B. The milk collection device 100 may comprise a cap 116, an example is depicted in Figure 5. The milk collection device 100 may comprise a non-return valve (NRV) 114, an example is depicted in Figure 6. The milk collection device 100 comprises a diaphragm (obscured in Figure 1), an example is depicted in Figure 4A. Examples of these components in a milk collection device can be seen in Figure 14 in an exploded view.

Figures 2A and 2B depict a breast shield 200 according to an embodiment of the invention. Figure 2A is an isometric view. Figure 2B is a two-dimensional (2D)cross-sectional view. The dashed line in Figure 2A shows the plane in which the cross sectional view shown in Figure 2B is taken.

The breast shield 200 comprises a nipple tunnel 210 and a breast flange 216. In use, the breast flange contacts the user's breast and the nipple tunnel houses the user's nipple. The breast flange 216 may be convex in order to fit the shape of a breast. The nipple tunnel 210 may be substantially cylindrical. The nipple tunnel 210 may be a cylindrical annulus shape. The nipple tunnel 210 is shaped to receive a nipple. The nipple tunnel 210 extends in the direction that a nipple is inserted in use of the device.

The breast shield 200 comprises an outer rim portion 214. The breast flange 216 comprises the outer rim portion 214. The outer rim portion 214 is the outermost section of the breast flange. That is, the portion of the breast flange with a larger circumference. The outer rim portion 214 may include a seal portion 212. This seal portion 212 is configured for sealing the breast shield 200 to the shell (for example the shell depicted in Figures 3A and 3B). When the breast shield 200 is attached to the shell, a seal is formed between the breast shield and shell, said seal may be referred to as the breast shield to shell external seal. The breast shield is removably attachable to the shell and when attached, the breast shield 200 and shell form a milk container. The breast shield may be removably attachable to the circumference of the shell. That is, the interior volume between the shell and the breast shield forms a milk container. The milk container is a chamber in which milk is collected.

As can be seen in Figure 2B, the material of the breast shield 200 may be thicker at the nipple tunnel 210 than at the breast flange 216. In some examples the material of the breast shield 200 is thicker at the nipple tunnel 210 than at the outer rim portion 214. The thicker the material, the less it is able to distort. In other words, the more rigid the nipple tunnel becomes. Therefore, in this example, distortion is controlled and there is less distortion at the nipple tunnel. When this feature is combined with a rigid supporting member, described in detail below, the nipple tunnel's rigidity during use is improved which results in improved milk pumping efficacy. The advantage of a thinner material at the breast flange is for ease of attaching the breast shield to the shell and for improved comfort of the breast flange on the breast during use.

Figures 3A and 3B depict a shell 300 according to an embodiment of the invention. Figure 3A is an isometric view. Figure 3B is a 2D cross-sectional view. The dashed line in Figure 3A shows the plane in which the cross sectional view shown in Figure 3B is taken.

The shell comprises a substantially hemispherical section. The shell 300 comprises an external side 310 and an internal side 320. The external side 310 is the exterior surface/s of the shell 300. The external side 310 comprises a curved portion 350 and an end region 360. On the opposing side of the shell 300 to the end region 360 is an open-ended side 340.

The breast shield is removably attachable to the shell 300 at the open-ended side. The diaphragm is removably attachable to the shell 300 at the external side 310. Specifically, in this example, at the end region 360 of the external side 310. The shell is rigid.

The shell 300 may be a substantially hemispherical ring shape. The inner radius of the ring defines the internal side 320. The remaining surfaces define the external side 310.

In addition to the substantially hemispherical section, the shell 300 may also comprise a rigid supporting member 330 for supporting the nipple tunnel. The rigid supporting member 330 may be integrally formed with or fixedly attached to the hemispherical section. The example shown in Figures 3A and 3B is where the rigid supporting member 330 is integrally formed.

The rigid supporting member 330 may be shaped like the breast shield nipple tunnel. The rigid supporting member 300 and/or nipple tunnel may be a cylindrical annulus. The rigid supporting member may comprise a cylindrical and/or conical region. That is, the shape of the rigid supporting member 300 may be entirely cylindrical, entirely conical or comprise a cylindrical and conical region. The cylindrical region may be a similar shape to the nipple tunnel. The conical region may be a similar shape to the base of the breast flange. The rigid supporting member 330 may be trumpet shaped. A trumpet shape may be defined as a truncated cone shape.

The rigid supporting member 330 may be configured to support the shape of the nipple tunnel. Especially, when the nipple tunnel is deformable if, for example, the nipple tunnel is made from a deformable material. When the breast shield and shell are attached, the rigid supporting member 330 may be configured to support the nipple tunnel of the breast shield. The rigid supporting member 330 may be configured to contact the nipple tunnel when the breast shield and shell are attached.

The rigid supporting member 330 may be configured to support the breast shield. In some embodiments, the cylindrical region is configured to support the breast flange and the conical region to support the nipple tunnel. The rigid supporting member 330 may be configured to support the nipple tunnel of the breast shield when the device is in use. More generally, the rigid supporting member 330 may be configured to support the user's breast and/or nipple when the device is in use.

The rigid supporting member 330 may support the nipple tunnel during use of the device. During use, the nipple tunnel may deform, this deformation is controlled and/or prevented by the rigid supporting member 330. This has the benefit of improving milk production efficacy. The rigid supporting member provides resistance to the breast tissue for improved milk extraction. In particular, providing resistance around the nipple and surrounding breast tissue close to the nipple. The combination of a rigid supporting member and a deformable, flexible, breast shield provides a comfortable device with a high milk production efficacy.

The rigid supporting member 330 may form the internal side 320 of the shell 300. The rigid supporting member 330 may be made from any suitably rigid material, suitable for supporting the nipple tunnel.

Figure 4A depicts a diaphragm 400 according to an embodiment of the invention. The diaphragm 400 comprises a dry side 410 for connection to an air pump, such as the air pump 120 in Figure 1. The connection to the air pump may be via an airline, such as the air-line 118 in Figure 1. The connection to the air pump and/or air-line may be via a cap, such as the cap 116 in Figure 1 or Figure 5.

The diaphragm 400 is a barrier between the milk and the air pump, preventing the milk from reaching the dry side of the and therefore from reaching the air-line or air pump. The dry side 410 is configured to contact air and not milk. The wet side 420 is configured to contact milk and/or air.

The diaphragm 400 may be substantially circular. In some embodiments, the diaphragm is an oval shape. In Figure 4 the diaphragm 400 is a flexible membrane. The diaphragm 400 may be made from any suitable flexible material, such as a suitable silicone.

In some embodiments, the diaphragm 400 comprises a seal 430. The seal 430 is formed between the diaphragm 400 and shell. The seal 430 may be formed between the diaphragm 400 and the external side of the shell.

The seal 430 may be circumferential to diaphragm. That is, the seal is present across the entire circumference of the diaphragm. The seal 430 may be homogeneous across the circumference.

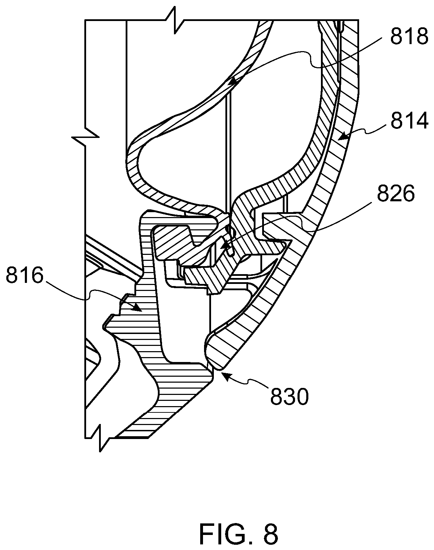

The seal 430 is configured such that in order for milk to overcome the seal, milk must flow in two opposite parallel directions. This feature is explained below with reference to Figure 8.

The seal 430 may be an interference seal. That is, the seal 430 is configured for an interference fit between diaphragm 400 and the shell. The seal 430 may comprise one or more lip seal elements.

When the milk collection device is assembled for use, the diaphragm 400 is affixed over the end of the nipple tunnel, such that the diaphragm seals the end of the nipple tunnel. Therefore, no part of the shell is blocking the space between diaphragm 400 and nipple tunnel. Accordingly, the space for receiving the nipple is longer than in a device in which the shell is positioned between the nipple tunnel and the diaphragm. This allows for facilitation of a longer nipple. This is advantageous as the user's nipple elongates during use of the breast pump. The device is suitable for users with longer nipples. In effect, the device provides a longer nipple tunnel without having to increase the other dimensions of the device (for example the profile of the device when worn by the user). Therefore the device is discrete whilst being suitable for longer nipples.

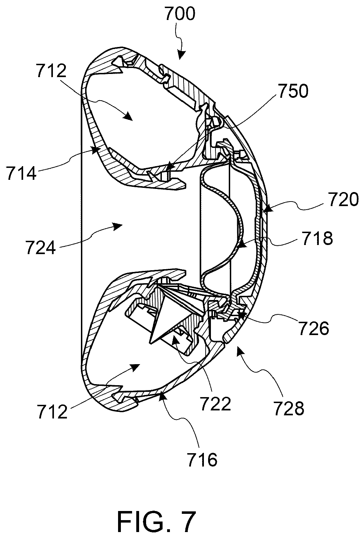

The shape of the diaphragm may also allow for facilitation of a longer nipple in the nipple tunnel. In these examples, the diaphragm is dome shaped. A dome shape has a dome-like or hemispherical projection. The height of the dome lengthens the nipple tunnel. This can be seen in Figure 7 in the assembled device. Other diaphragms which do not extend the nipple tunnel may also be used, for example, a flattened disc shape diaphragm. It is not essential that the diaphragm be dome shaped to facilitate a longer nipple in the nipple tunnel, for example other shape diaphragms which accommodate a longer nipple may be used.

When the milk collection device is assembled for use, the diaphragm 400 may be co-axial to the nipple tunnel, where the axis runs through the nipple tunnel parallel to the nipple when the nipple is inserted into the nipple tunnel.

The diaphragm 400 may be perpendicular to the nipple tunnel. In other words, the diameter of the diaphragm 400 is perpendicular to the nipple tunnel in the direction of nipple insertion. Figure 7 shows an example where the diaphragm is perpendicular to the nipple tunnel. In other examples, the diaphragm is not perpendicular to the nipple tunnel, for example 60, 70 or 80 degrees.

In other examples, the diaphragm 400 is not perpendicular to the nipple tunnel. For example, the angle between the diaphragm 400 and nipple tunnel is between 30 and 80 degrees, where the angle is measured between the length of the nipple tunnel, in the direction of nipple insertion, and the diameter of the outer circumference of the diaphragm.

Figure 4B shows an assembled milk collection device comprising a diaphragm 400,a bung 440 attached to the diaphragm, a shell 460, a cap 470, a breast shield 480 and a non-return valve 490. The shell 460 comprises a decanting opening 450 configured to pour milk from the milk container, the milk container formed from the shell 460 and breast shield 480. In other words, the decanting opening 450 allows milk to be decanted from the device.

In the device shown in Figures 4A and 4B the bung 440 is attached to the diaphragm. In other examples, the bung 440 is not attached to the diaphragm.

The decanting opening 450 may be on the upper side of the shell 460, when the device is orientated for use. The bung 440 may be configured to fit into the decanting opening 450. The bung 440 may be configured to seal the decanting opening 450. The bung 440 may pivot in and out of the decanting opening 450, as shown by the arrow. The pivot point is created by the shell 460. The diaphragm is attached to the bung 440 via a hinge 442. In other words, the diaphragm is hingeably attached to the bung.

Optionally, the diaphragm 400, hinge 442 and bung 440 form an integral, monolithic, component. In other words, the diaphragm 400 and bung 440 form an integral component and are one piece. This has the benefit of reducing the number of total parts in the device. This improves ease of cleaning of the device and therefore device hygiene over time.

Optionally, the diaphragm 400, hinge 442 and bung 440 are configured such that when the user removes the bung 440 from the shell in order to decant the milk, the bung's neutral position is away from the flow of milk. In some examples, the direction and shape of the hinge 442 is configured to hold the bung 440 away from the decanting opening 450. That is, when not housed in the external surface of the shell of the device (i.e. in an 'open' position) the neutral position of the bung is away from the flow of the milk. This means the user can decant milk from the device without the bung getting in the way of the flow of milk. This makes for an easier and cleaner milk decanting process.

The bung 440 is movable between an open and closed position.The bung 440 has the closed position when secured within the decanting opening and the open position when not secured within the decanting opening. When in the open position, the hinge between the bung and the diaphragm biases the bung into a neutral position away from the flow of the milk. The bung pivots between the closed and open position via the hinge. In the neutral position, in some examples the plane of the bung is at an angle of greater than 30 degrees relative to the plane of the decanting opening or an angle of greater than 45 degrees to the plane of the decanting opening . In some examples, in the neutral position the plane of the bung is at an angle of greater than 70 or 80 degrees to the plane of the decanting opening. By being positioned away from the decanting opening in the neutral position, for example by an angle of greater than 70, 75 or 80 degrees, the bung is positioned out of the flow of the milk when being decanted from the device. In some examples, in the neutral position the plane of the bung is at an angle of substantially 90 degrees to the plane of the opening, or greater than 90 degrees to the plane of the decanting opening.

In some examples the hinge biases the bung 440 into a neutral position in which the bung is not in the same plane as the diaphragm. This can be seen in Figure 4B. The plane of the diaphragm in Figure 4B is vertical. The plane of the diaphragm is the same plane as the diameter of the diaphragm.

In some examples, the bung is not attached to the diaphragm. The bung is attached (fixedly or removably attached) to the shell via a hinge. The bung and hinge perform the functions of the bung and hinge described above. That is, the bung is movable between an open and closed position. When the user removes the bung from the shell in order to decant the milk, the bung's neutral position is away from the flow of milk. The direction and shape of the hinge is configured to hold the bung away from the decanting opening. When in the open position, the hinge between the bung and the diaphragm biases the bung into a neutral position away from the flow of the milk. The bung pivots between the closed and open position via the hinge. When in the open position, the hinge between the bung and the diaphragm biases the bung into a neutral position away from the flow of the milk. In the neutral position, in some examples the plane of the bung is at an angle of greater than 30 degrees relative to the plane of the decanting opening or an angle of greater than 45 degrees to the plane of the decanting opening . In some examples, in the neutral position the plane of the bung is at an angle of greater than 70 or 80 degrees to the plane of the decanting opening. By being positioned away from the decanting opening in the neutral position, for example by an angle of greater than 70, 75 or 80 degrees, the bung is positioned out of the flow of the milk when being decanted from the device. In some examples, in the neutral position the plane of the bung is at an angle of substantially 90 degrees to the plane of the opening, or greater than 90 degrees to the plane of the decanting opening.

Optionally, the bung 440 and decanting opening 450 are configured to form a continuous surface of the shell when the bung is sealing the decanting opening 450. The decanting opening 450 is configured to accommodate the bung 440 such that the other surface of the shell is continuous when the bung seals the opening. In other words, the bung 440 and shell 460 are configured to form a flush outer surface. This improves discretion since the device can more readily be worn in a bra.

Figure 4A shows the diaphragm and the bung 440 when not assembled as part of the device. The bung is in the neutral position. As can be seen, the bung when viewed in cross section is not in the same plane as the diaphragm. The plane of the diaphragm in Figure 4A is vertical. The plane of the diaphragm is the same plane as the diameter of the diaphragm. The hinge biases the bung into a neutral position in which the bung is not in the same plane as the diaphragm. In the neutral position, there is an angle between the plane of the bung and the plane of the diaphragm (the plane of the bung intersects with the plane of the diaphragm at a pivot point on the hinge). The angle between the plane of the diaphragm and the plane of the plane of the bung is between 5 and 50 degrees, where the wet side of the bung is angled away from the wet side of the diaphragm. In other words, there is a reflex angle between the wet side of the diaphragm and the wet side of the bung.

Figure 5 depicts a cap 500 according to an embodiment of the invention. Figure 5 is an isometric view.

The cap 500 may be removably attachable to the dry side of the diaphragm and/or the shell. The cap 500 may be connectable to the air pump. The cap 500 may be connectable to the air pump via an air-line.

The cap 500 may be a hemispherical shape. The cap 500 may comprise a flat portion 510. The flat portion 510 allows for the cap 500 to sit flat on a surface. Therefore, when the milk collection device is assembled, the flat portion 510 allows the entire device to sit flat on a surface, with the nipple tunnel extending vertically.

Figure 6 depicts a non-return valve (NRV) 600 according to an embodiment of the invention. Figure 6 is an isometric view.

The NRV enables milk to pass in one direction. That is, from the nipple tunnel and into the milk container. The milk container is formed from the breast shield and shell attached to each other.

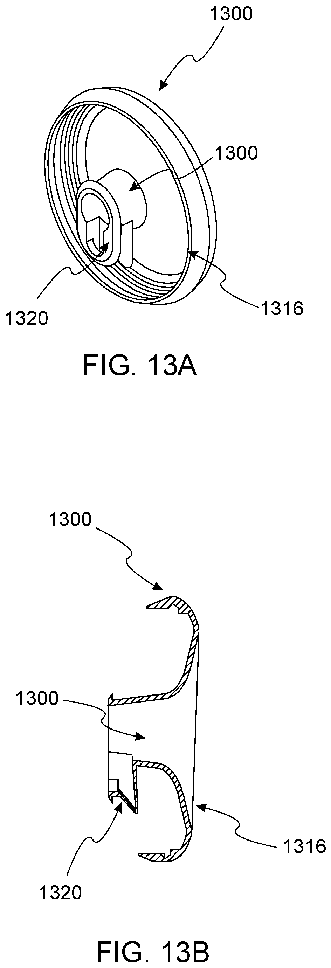

In some embodiments, the NRV and breast shield may be one piece, as shown in Figures 13A and 13B. In other words, the NRV and breast shield are monolithic. This has the advantage of a reduction in the number of washable and small parts. In other embodiments, the NRV 600 is separate to the breast shield, such an NRV is shown on Figure 6.

Figure 7 shows an assembled, ready for use, milk collection device 700 according to an embodiment of the invention. Figure 7 is a cross-sectional view. The components shown in Figures 2A to 6 may attach to each other to form the milk collection device 700.

The milk collection device 700 may comprise a breast shield 714, a shell 716, a diaphragm 718, a cap 720 and an NRV 722. Each of these components may have features as described above with reference to Figures 2A to 6. The breast shield 714 may comprise a nipple tunnel 724 for receiving a nipple (as shown).

As explained with reference to the shell in Figure 3, the shell 716 shown in Figure 7 may comprise a rigid supporting member 750. The rigid supporting member 750 may be configured to support the shape of the nipple tunnel. The rigid supporting member may comprise a cylindrical and/or conical region. The rigid supporting member 750 may be trumpet shaped. This can be seen in Figure 7, the rigid supporting member 750 supports the nipple tunnel and part of the breast flange, the part of the breast flange closest to the nipple tunnel. This rigidity helps to improve milk pumping efficacy.

The breast shield 714 is removably attachable to the shell 716 and when attached, the breast shield and shell form a milk container 712. That is, the breast shield 714 and shell 716 when attached form a milk container therebetween. The shape of the milk container 712 may be an annulus. The milk container 712 may surround the nipple tunnel. The milk container 712 fills with milk during use of the device.

As mentioned with reference to Figures 2A and 2B, the breast shield may be deformable. The deformable breast shield supported by the rigid supporting member 750 provides user comfort from the soft cushioning of the breast shield. In some embodiments, there is a rigid region around the nipple to ensure sufficient and targeted resistance to the breast tissue, which maximises milk output. In particular, resistance is provided to the nipple and the breast tissue close to the nipple. Therefore, both improved comfort and milk efficacy can be provided together.

The path of the milk during use of the device, i.e., the milk flow path, starts at in the nipple tunnel 724 as milk is extracted from the breast. The milk flows from the nipple tunnel 724 through the NRV 722 into the milk container 712. The milk may contact the wet side of the diaphragm 718.

As explained above, the dry side of the milk collection device or breast pump system comprises the parts which do not come into contact with milk during normal use. The wet side of the milk collection device or breast pump system comprises the parts which do come into contact with milk during normal use.

In the milk collection device, the dry side comprises the cap and the space between the dry side of the diaphragm and the cap. In the breast pump system, the dry side further comprises the air-lines and air pump. In the milk collection device or breast pump system, the wet side comprises the nipple tunnel, wet side of the diaphragm, NRV and milk container.

The diaphragm 718 may be removably attachable to the shell 716 and when attached, a seal 726 is formed between the diaphragm and the external side of the shell. Similarly, when the milk container 712 is assembled, the diaphragm 718 may be configured to seal over the external side of the milk container 712. The external side of the milk container 712 is the outermost side/s of the milk container. The internal side of the milk container 712 contacts the milk as the milk fills the milk container during use.

Known devices comprise a seal between the diaphragm and an internal side of the shell or milk container. This means that for such known devices there is sometimes only one seal between the milk container and the dry side of the system (i.e., leading to the air-line and air pump). Since the seal of known devices is on the internal side of the shell, the seal contacts the milk which is collecting within the milk container. These known configurations can allow milk to reach the dry side and therefore the air-lines. Milk present in the air-lines can interfere with the effectiveness of the vacuum transferred from the air pump. Cleaning the air-lines can be difficult and require specialist cleaning equipment which is not readily available to users at home. Therefore, milk can build up in the air-lines over time.

In the present disclosure, the diaphragm 718 may be configured to seal over the external side of the milk container 712. This configuration has the advantage that the milk contacting the seal is not the milk contained in the milk container 712 but instead the milk present in the nipple tunnel 724. Since milk is only transient in the nipple tunnel, this configuration has advantages. Firstly, less milk contacts the seal 726 overall during use and therefore the risk of milk overcoming the seal is reduced. Secondly, the seal 726 is subject to less stress since the milk is not constantly pressing against the seal 726, making it less likely for the milk to overcome the seal. The only milk paths from the milk container 712 to the dry side of the diaphragm 718 are via an external side of the shell.

Therefore, the configuration of the seal 726 over the external side of the milk container 712 prevents milk reaching the dry side and therefore the air-lines and air pump. This has the effect of maintaining the effectiveness of pumping and the lifetime of the breast pump system. This configuration also allows for further features to be added in order to further prevent milk from reaching the air-lines and air pump, as will now be described.

The seal 726 may be circumferential to the shell 716 and diaphragm 718. The seal 726 may be configured such that in order for milk to overcome the seal, milk must flow in two opposite parallel directions. For example, the seal 726 may comprise a seal between the diaphragm and external side of the milk container and a seal between the diaphragm and cap 720. The seal 726 is configured such that even if the seal between the diaphragm and external side of the milk container is overcome, the milk must then also overcome a seal between the diaphragm and the cap, in order to get to the dry side and into the air-line. The seal 726 is configured such that the path milk has to follow in order to reach the dry side is in two opposite parallel directions. When the device is orientated for use, this means that milk has to travel against the direction of gravity. Thus, further preventing milk reaching the air-lines.

Therefore, in other words, the seal 726 may be configured such that in order for milk to flow from the dry side to the wet side the milk must flow against the direction of gravity.

Another optional feature which prevents milk reaching the air-line is an opening 728 formed between the shell 716 and cap 720. This feature is exemplified in further detail in Figure 8.

Figure 8 shows part of an assembled, ready for use, milk collection device according to an embodiment of the invention. Figure 8 is focused on the seal 826 between the diaphragm 818, shell 816 and cap 814.

An opening 830 may be formed between the cap 814 and the shell 816 on the lower half of the milk collection device when the device is orientated for use, such that milk which overcomes the seal will exit the device via the opening following gravity.

Since milk is immediately expelled from the device via the opening 830 following gravity, it is exceedingly difficult for milk to travel towards the dry side of the device. Therefore, this feature prevents milk reaching the dry side and the air-line.

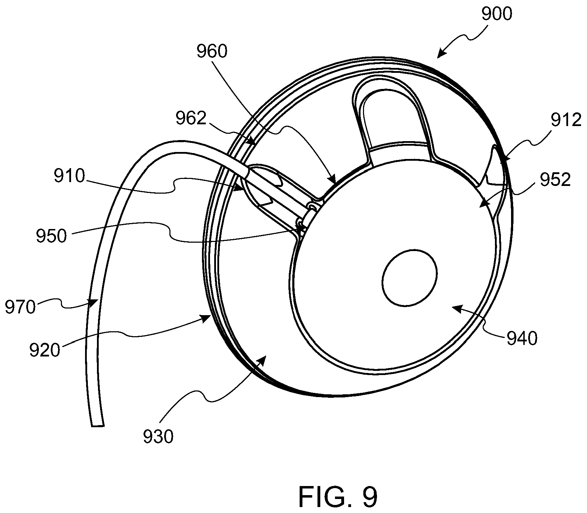

Figure 9 shows an assembled, ready for use, milk collection device 900 according to an embodiment of the invention. The milk collection device 900 comprises a breast shield 920, a shell 930 and a cap 940. The breast shield 920, shell 930 and cap 940 are removably attachable to each other according to any of the embodiments described herein.

The shell 930 comprises a first recess 910 and second recess 912. Each recess is configured to house an air-line 970. Each recess is configured to guide the air-line 970 into the milk collection device 900. This configuration allows the air-line 970 to contour to the shape of the milk collection device and is guided in a certain direction. This means that the air-line 970 is subjected to less bending over its lifetime. This reduces the stress on the air-line 970 and increases its lifetime. Furthermore, bends in the air-line 970 restrict the cross section of the air-line and inhibits the transfer of vacuum to the device. Therefore, according to the present disclosure, the effectiveness of the pump is improved.

The first recess 910 and second recess 912 being present in the shell 930, rather than in another location such as the cap 940, provides a more discreet profile for the device when worn in-bra. Features such as recesses create an unnatural profile to the device which is visible when worn in-bra. When the recesses are set back, such as in the shell, the profile is less disrupted and provides improved discretion in-bra.

The cap 940 may comprise an opening 950 connectable to the first 910 and second recesses 912. When cap 940 is attached to the diaphragm, the opening 950 is connected to either the first 910 or second 912 recess. The cap 940 is rotatable such that user can align the opening 950 with either the first 910 or second recesses 912. When the opening 950 is connected to the one recess, the other recess may be closed off by the cap.

The device is configured such that the air-line 970 is attachable to the first opening 950 or the second opening 952. This is such that the air-line 970 is able to connect via the first or second opening to the dry side of the diaphragm / device.

In other embodiments, the cap 940 may comprise a first opening 950 and a second opening 952 connectable to the first 910 and second recesses 912 respectively. The device may be configured such that the air-line 970 is attachable to the first opening 950 or the second opening 952. This is such that the air-line 970 is able to connect via the first or second opening to the dry side of the diaphragm / device.

Each recess is configured such that, when no air-line 970 is present, a user can use the recess to remove cap from the rest of the device. The recess allows the user to create more force on the cap in order to break the seal between the cap and the diaphragm. For example, one air-line 970 can be housed in one recess whilst the other recess is used to remove the cap from the rest of the device.

In other embodiments, only one recess and one corresponding opening is present in the device.

The breast shield 920, shell 930 and cap 940 may be configured to form a curved external surface when the breast shield, shell and cap are attached to each other. The curved external surface may be hemispherical. This is to create a breast-like profile for discretion using the device in-bra.

The device may be configured such that the depth of the cap 940 makes up at least a quarter of the total depth of the assembled device. In another example, the depth of the cap 940 makes up at least a third of the total depth of the assembled device. The depth is defined as the distance from the front face of the device to the breast flange. The depth may be defined along the axis parallel to the nipple and nipple tunnel.

The device may be configured such that the cap 940 makes up at least a third of the total surface area of the external surface. The total surface area is the total surface area of the milk collection device when assembled.

The cap 940 therefore may have a significant size compared to the other components. This means that the first 910 and second recesses 912 are set back from the front face of the device, since the recesses are in the shell. The front face of the device is the direction facing away from the user when the device is orientated for use. The front face of the device may be defined by the cap 940. When the recesses 910, 912 are set back from the front face of the device, the profile of the device is more breast-like and therefore improves discretion of the device when worn in-bra.

The shell 930 comprises an innermost circumference 960 for connection to the cap 940; and an outermost circumference 962 for connection to the breast shield 920. That is, the innermost and outermost circumferences are approximately the location for connection to the cap and breast shield respectively.

The first 910 and second 912 recesses may extend from the innermost circumference 960 in a perpendicular direction towards the outermost circumference. That is, each recess may have one end which is at the innermost circumference 960, therefore creating a break in the innermost circumference. Each recess may end part way through the surface of the shell. The first 910 and second 912 recesses may be elongate in shape.

The corresponding first opening 950 and second opening 952 align with the first 910 and second 912 recesses. Therefore, the first opening 950 and second opening 952 may be perpendicular to the innermost circumference 960 of the shell 930, when the device is assembled. This configuration guides the air-line 970 into the shell 930 in a direction perpendicular to the circumference of the shell 930.

Known devices have configurations leading air-lines to be tangential to the circumference of the breast shield, shell, or cap. The perpendicular configuration of the present disclosure reduces the amount of bending of the air-lines which has the benefits described above.

The first recess 910 may be at a separate location along the innermost circumference 960 to the second recess 912. In some examples, the distance between the first recess 910 and second recess 912 along the innermost circumference 960 is at least a third of the innermost circumference. In some examples, the first recess 910 and second recess 912 are diametrically opposed. As described above, one recess may be used for guiding an air-line 970 whilst the other air-line is used to remove the cap 940 from the rest of the device. The recesses at separate locations around the circumference improves the ease in which both functions can occur without interfering with each other.

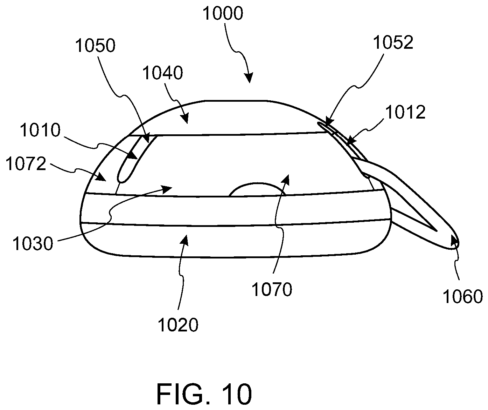

Figure 10 shows an assembled, ready for use, milk collection device 1000 according to an embodiment of the invention. The milk collection device 1000 comprises a breast shield 1020, a shell 1030 and a cap 1040. The breast shield 1020, shell 1030 and cap 1040 are removably attachable to each other according to any of the embodiments described herein.

Figure 10 shows the milk collection device 1000 top down from the view of the user with the device ready for use, in contact with their breast.

Similarly to the milk collection device 900 depicted in Figure 9, the shell 1030 comprises a first recess 1010 and second recess 1012. The cap 1040 may comprise a first opening 1050 and a second opening 1052 connectable to the first 1010 and second 1012 recesses respectively.

An air-line 1060 is guided by the second recess 1012 and connected to the second opening 1052. The air-line 1060 is connected to the dry side of the device, ready for use.

The shell 1030 may comprise a first section 1070 and a second section 1072, where the first section is transparent, and the second section is transparent. In some examples, the second section is opaque. The first section 1070 is transparent in order to allow the user to view inside the device, either to aid in nipple and/or breast alignment or to view the milk level collecting in the milk container.

The first 1010 and second recesses 1012 may form boundaries between the first 1070 and second sections 1072 of the shell. In some examples, the recesses are sufficiently far apart, for example a least a third of the circumference, such that a sufficient transparent first section 1070 is defined, which acts as a viewing window for the user to aid in nipple and/or breast alignment.

Preferably, the first section 1070 is devoid of openings or attachments. In other words, there are preferably no divots, dents or air-lines attached to this section. Preferably, the first section 1070 has reduced visual distortion to allow for a clear view into the device.

The first section 1070 may be above the second section 1072 when the device is orientated for use. Therefore, the first section 1070 can be used as a viewing window by the user when the device is contacting the breast ready for use, to aid nipple and/or breast alignment.

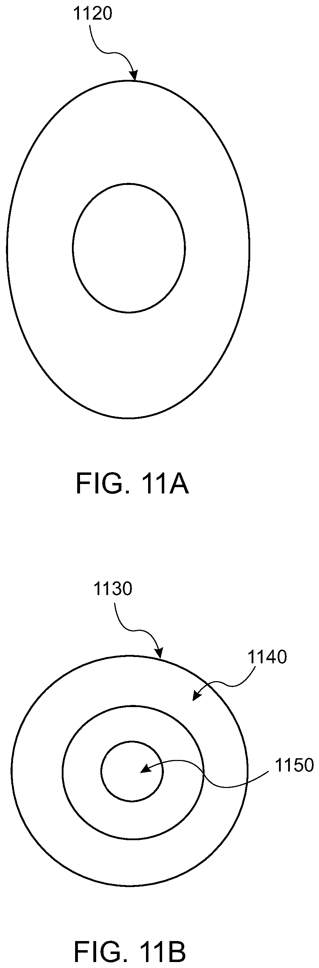

Figure 11A depicts a shell 1120 according to an embodiment of the invention. Figure 11B depicts a breast shield 1130 according to an embodiment of the invention. Figures 11A and 11B are 2D views.

As described previously, the breast shield 1130 is removably attachable to the circumference of the shell 1120 and when attached, the breast shield and shell form a milk container. The breast shield 1130 may be made from a deformable material such that the breast shield is configured to deform to attach to the shell 1120. In order to attach the breast shield 1130, a user deforms the breast shield and stretches it over the shell. The shell may comprise sealing elements around the circumference such as lips to aid the attachment. The shell 1120 may be made from a non-deformable material. The breast shield may be made of any suitable material, such as silicone.

The breast shield 1130 may be attachable to the shell 1120 in any orientation of the breast shield. That is, the device is configured such that when the circumferences of breast shield 1130 and shell 1120 are parallel to each other, the breast shield can attach to the shell when the breast shield is rotated in this plane at any degree.

Known devices require a specific orientation of breast shield relative to other components of the device, which means the user can misalign the breast shield resulting in below optimal use of the breast pump. The present disclosure removes the need for specific relative alignment of the breast shield to the components, improving effectiveness of the device and reducing the user's time spent trying to align the breast shield.

The circumference of the breast shield 1130 and circumference of the shell 120 may be different shapes. For example, the circumference of the breast shield 1130 (when not attached to the shell) may be circular and the circumference of the shell 120 an oval shape. The advantage of a non-circular shell 120 is that the device's shape more-closely resembles that of a breast and the device therefore sits more discreetly under a bra. This is compared to a circular shell shape which is a less breast-like shape and less discrete.

It will be understood that real breasts take many shapes, forms, and sizes, and whilst the oval shape in combination with the circular breast shield has been shown in the figures, other non-circular shells may be used that may sit more discreetly under a bra taking other shapes, forms, or sizes. Users may be more likely to engage with a pump which is more discreet and/or looks good in use.

By having a circular breast shield, the breast shield can be attached, for example stretched onto, and positioned over, the shell in any orientation. Because the breast shield is deformable, once it is attached to the shell, which is rigid, it conforms to the shape of the shell. Therefore, in the assembled configuration, the device retains the shape of the shell.

The shell circumference to which the breast shield is attached has a discrete order of rotational symmetry, that is, the shell circumference does not have the same shape at all angles of rotation. In some examples, the shell circumference has an order of rotational symmetry of 1 or 2. In contrast, the breast shield circumference, when not attached to the shell, has an infinite order of rotational symmetry, meaning the shape is the same at all angles of rotation.

This means that, when a user is faced with the task of attaching the breast shield to the shell, the breast shield can be positioned on the shell at any orientation/ angle of rotation, since the shape is the same at all angles of rotation. This removes the need for the user to spend time finding the correct orientation of the breast shield before attaching it to the shell.

Similarly to Figures 2A, 2B, the breast shield 1130 comprises a nipple tunnel 21150 and an outer rim portion 1140. The breast flange comprises the outer rim portion 1140. The outer rim portion 1140 is the outermost section of the breast flange. That is, the portion of the breast flange with the larger circumference. The outer rim portion 1140 may include a seal portion.

Deformation of the breast shield 1130 to attach to the shell 1120 results in distortion of the breast shield since the material is stretched. It is advantageous to control distortion for optimal use of the milk collection device. As such, the material of the breast shield 1130 may be thicker at the nipple tunnel 1150 than at the breast flange. In some examples the material of the breast shield 1130 is thicker at the nipple tunnel 1150 than at the outer rim portion 1140. The thicker the material, the less it is able to distort. In other words, the more rigid the nipple tunnel becomes. Therefore, in this example, distortion is controlled and there is less distortion at the nipple tunnel. There is more distortion at the outer rim portion. Therefore, the nipple tunnel retains its shape, allowing it to retain its shape suitable for use with the rest of the device. For example, it retains its shape suitable for receiving a nipple and for connection to the diaphragm. The distortion at the outer rim portion is less important, deformation can occur and the breast shield will still have a good fit onto the breast.

In some embodiments, the surface of the breast shield can be textured which allows a better fit onto a breast, due to an increased contact surface.

Since the breast shield 1130 is made from a deformable material, when the breast shield and shell are attached, the shell may be configured to support the shape of the nipple tunnel. For example, the shell 1120 may comprise a rigid supporting member. The rigid supporting member may be shaped similarly to the nipple tunnel. The rigid supporting member may comprise a cylindrical and/or conical region. The rigid supporting member may be trumpet shaped. The rigid supporting member contacts the nipple tunnel 1150, this prevents the nipple tunnel 1150 from deforming out of shape.

The rigid supporting member may be configured to support the shape of the nipple tunnel. When the breast shield and shell are attached, the rigid supporting member may be configured to support the nipple tunnel of the breast shield. The rigid supporting member may be configured to contact the nipple tunnel when the breast shield and shell are attached.

The rigid supporting member has the benefit that it improves the efficacy of milk extraction. The combination of a rigid supporting member and a deformable, flexible, breast shield provides a comfortable device with high milk production efficacy. These features of the breast shield and shell can be applied to any of the milk collection devices described herein.

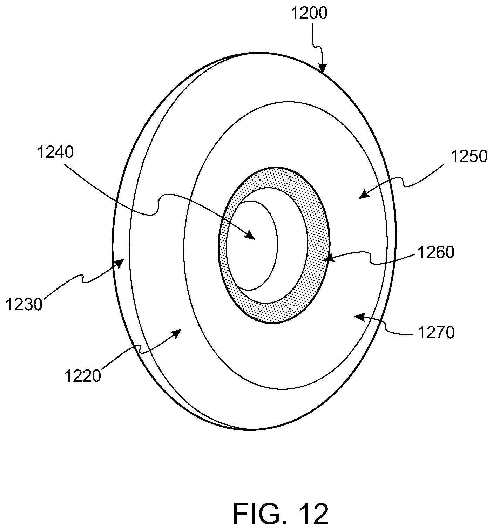

Figure 12 shows an assembled, ready for use, milk collection device 1200 according to an embodiment of the invention. The milk collection device 1200 comprises a breast shield 1220 and a shell 1230. The breast shield 1220 and shell 1230 are removably attachable to each other according to any of the embodiments described herein.

The breast shield 1220 may comprises a nipple tunnel 1240 and a breast flange 1250 for contact with a user's breast. The breast flange 1250 may comprise the entire section of the breast shield 1220 configured to contact the user's breast. The breast flange 1250 may comprise an inner annular portion 1260 and an outer annular portion 1270. The inner annular portion 1260 has a smaller radius compared to the outer annular portion 1270.

It is advantageous for the breast flange to have good retention and stability on the breast. As such, in some embodiments, the surface smoothness of the inner annular portion 1260 and the outer annular portion 1270 are different. Having two different surface textures on the breast flange 1250 improves retention and stability on the breast.

The inner annular portion 1260 may have a smaller surface area compared to the outer annular portion 1270. An example of these proportions is shown in Figure 12.

In some embodiments, the inner annular portion 1260 has a higher surface smoothness compared to the outer annular portion 1270.

When the inner annular portion 1260 has a smaller surface area and a higher surface smoothness compared to the outer annular portion 1270, this provides reduction the size of the higher surface smoothness area. This is advantageous as a higher surface smoothness area attracts dust and limescale build up due to use over time.

In other embodiments, the inner annular portion 1260 has a higher surface smoothness compared to the outer annular portion 1270.

The inner annular portion may have a surface finish SPI-A1, SPI-A2 or SPI-A3. The SPI Finish refers to the surface finish standard set by SPI (Society of the Plastics Industry).

Figures 13A and 13B depict a breast shield 1300 according to an embodiment of the invention. Figure 13A is an isometric view. Figure 13B is a 2D cross-sectional view.

The breast shield 1300 comprises a nipple tunnel 1310, breast flange 1316 and NRV 1320. In this embodiment, the nipple tunnel 1310, breast flange 1316 and NRV 1320 are integrally formed, that is they are monolithic . That is, they are one piece. This has the advantage of reducing the total number of parts in the device, therefore improving ease of cleaning and hygiene of the device.

In other examples, as explained above, the NRV is not attached to or integrally formed with the breast shield.

Figure 14 depicts an exploded view of example components of a milk collection device. The exploded milk collection device comprises: a cap 1410, a diaphragm 1420, a shell 1430 and a breast shield 1440. As described with respect to previous Figures, the breast shield 1440 may attach to the shell 1430 to form a milk container therebetween. The shell 1430 may attach to the diaphragm 1420, such that the diaphragm 1420 seals the end of the nipple tunnel. The diaphragm 1420 may attach to the cap 1410. The cap 1410 may attach to an external air-pump via an air-line, as depicted in Figure 1.

Unless explicitly stated otherwise, the features of the milk collection devices and breast pump system can be combined in any suitable manner.

It will be understood that the above description is of specific embodiments by way of aspect only and that many modifications and alterations will be within the skilled person's reach and are intended to be covered by the scope of the appendant claims.

The following clauses are also provided as part of this disclosure. The features from the clauses may be provided in any conceivable combination.
1. An in-bra wearable milk collection device for use in a breast pump system, the milk collection device comprising:
   a breast shield comprising a nipple tunnel and breast flange;
   a shell;
   a diaphragm comprising a dry side for connection to an air pump and a wet side configured to contact milk;
   wherein the breast shield is removably attachable to the circumference of the shell and when attached, the breast shield and shell form a milk container.
2. The milk collection device of clause 1, wherein the diaphragm is removably attachable to the shell and when attached, a seal is formed between the diaphragm and an external side of the shell.
3. An in-bra wearable milk collection device for use in a breast pump system, the milk collection device comprising:
   a breast shield;
   a shell;
      wherein the breast shield is removably attachable to the shell and when attached, the breast shield and shell form a milk container;
   wherein the breast shield comprises a nipple tunnel and a breast flange for contact with a user's breast,
   and wherein the breast flange comprises an inner annular portion and an outer annular portion, wherein the surface smoothness of the inner annular portion and the outer annular portion are different.
4. An in-bra wearable milk collection device for use in a breast pump system, the milk collection device comprising:
   a breast shield;
   a shell;
   a cap;
   an air-line;
   a diaphragm comprising a dry side for connection to an air pump;
   wherein the breast shield is removably attachable to the shell and when attached, the breast shield and shell form a milk container.
   wherein the cap is removably attachable to the dry side of the diaphragm, and wherein the cap is connectable to the air pump via the air line,
   wherein the shell comprises a first and second recess, each configured to house the air line.
5. The milk collection device of any preceding clause, wherein the cap comprises an opening connectable to the first recess and the second recess, wherein when cap is attached to the diaphragm, the opening is connected to either the first or second recess.
6. The milk collection device of any preceding clause, wherein the breast shield, shell and cap are configured to form a curved external surface when the breast shield, shell and cap are attached to each other.
7. The milk collection device of clause 6, wherein the curved external surface is hemispherical.
8. The milk collection device of any preceding clause, wherein the shell comprises: an innermost circumference for connection to the cap; and an outermost circumference for connection to the breast shield.
9. The milk collection device of clause 8, wherein the first and second recesses extend from the innermost circumference in a perpendicular direction towards the outermost circumference.
10. The milk collection device of clause 8 or 9, wherein the first recess is at a separate location along the innermost circumference to the second recess.
11. The milk collection device of any of clauses 8 to 10, wherein the distance between the first recess and second recess along the innermost circumference is at least a third of the innermost circumference.
12. The milk collection device of any preceding clause, wherein the shell comprises: a first section; and a second section; wherein the first section is transparent and is devoid of attachments and/or openings.
13. The milk collection device of clause 12, wherein the first and second recesses form boundaries between the first and second sections.
14. The milk collection device of clause 12 or 13, wherein the first section is above the second section when the device is orientated for use.
15. The milk collection device of any preceding clause, wherein the shell is transparent.
16. The milk collection device of any preceding clause, wherein the device may be configured such that the depth of the cap makes up at least a quarter of the total depth of the assembled device, wherein the depth is defined as the distance from a front face of the cap to the breast shield.
17. The milk collection device of preceding clause, wherein the breast shield is made from a deformable material such that the breast shield is configured to deform to attach to the shell and wherein the breast shield is attachable to the shell in any orientation of the breast shield.
18. The milk collection device of any preceding clause, wherein the circumference of the breast shield and circumference of the shell are different shapes.
19. The milk collection device of clause 18, wherein the circumference of the breast shield has an infinite order of rotational symmetry, and the circumference of the shell has a discrete order of rotational symmetry; and/or
   wherein the circumference of the breast shield is circular and the circumference of the shell is an oval shape.
20. The milk collection device of any preceding clause, wherein the shell comprises a rigid supporting member.
21. The milk collection device of clause 20, wherein the rigid supporting member is configured to support the nipple tunnel when the breast shield and shell are attached and/or when the device is in use.
22. The milk collection device of clause 20 or 21, wherein the rigid supporting member is configured to support the user's breast and/or nipple when the device is in use.
23. The milk collection device of any of clause 20 to 22, wherein the rigid supporting member comprises a cylindrical annulus.
24. The milk collection device of any of claims 20 to 23 wherein the rigid supporting member comprises a conical region.
24. The milk collection device of any preceding clause, wherein the material of the breast shield is thicker at the nipple tunnel than at the breast flange.
25. The milk collection device of any preceding clause, wherein the breast shield comprises a nipple tunnel and an outer rim portion, wherein the material of the breast shield is thicker at the nipple tunnel than at the outer rim portion.
26. The milk collection device of any preceding clause, wherein the breast flange comprises an inner annular portion and an outer annular portion, wherein the surface smoothness of the inner annular portion and the outer annular portion are different.
27. The milk collection device of clause 26, wherein the inner annular portion has a higher surface smoothness compared to the outer annular portion.
28. The milk collection device of any preceding clause, wherein the inner annular portion has a surface finish SPI-A1, SPI-A2 or SPI-A3.
29. The milk collection device of any preceding clause, wherein the inner annular portion has a smaller surface area compared to the outer annular portion.
30. The milk collection device of any preceding clause, wherein the diaphragm seals the end of the nipple tunnel via a seal circumferential to the shell and diaphragm, wherein the seal is configured such that in order for milk to overcome the seal, milk must flow in two opposite parallel directions.
31. The milk collection device of any preceding clause, wherein the seal is an interference seal comprising one or more lip seal elements.
32. The milk collection device of any preceding claim, wherein when the milk collection device is assembled for use, the diaphragm is co-axial with the nipple tunnel.
33. The milk collection device of any preceding clause, wherein when the milk collection device is assembled for use, the diaphragm is perpendicular to the nipple tunnel.
34. The milk collection device of any preceding clause, wherein the device comprises a cap removably attachable to the diaphragm, wherein the cap is connectable to an air pump, and
   wherein an opening is formed between the cap and the shell on the lower half of the milk collection device when the device is orientated for use, such that milk which overcomes the seal will exit the device via the opening following gravity.
35. The milk collection device of any preceding clause, wherein the only milk paths from the milk container to the dry side of the diaphragm are via an external side of the shell.
36. The milk collection device of any preceding clause, wherein the shell comprises a decanting opening configured to pour milk from the milk container; and the diaphragm is attached to a bung via a hinge, wherein the bung is configured to seal the decanting opening.
37. The milk collection device of clause 36, wherein the diaphragm, hinge and bung form an integral component.
38. The milk collection device of clause 36 or 37, wherein the bung is movable between:
   a closed position wherein the bung is secured within the decanting opening; and
   an open position wherein the bung is not secured within the decanting opening.
39. The milk collection device of clause 38, wherein in the open position the hinge is configured to bias the bung into a neutral position in which the bung is positioned out of the flow of the decanting milk path, wherein the decanting milk path is the path milk flow from the device when being decanted via the decanting opening.
40. The milk collection device of clause 38 or 39 wherein in the open position the hinge is configured to bias the bung into a neutral position in which the bung is not in the same plane as the diaphragm.
41. The milk collection device of clause 38, 39 or 40, wherein in the open position the hinge is configured to bias the bung into a neutral position in which there is an angle of greater than 75 degrees between the plane of the decanting opening and the plane of the bung.
42. The milk collection device of any previous clause, wherein the shell comprises a decanting opening configured to pour milk from the milk container; and the device comprises a bung to the shell via a hinge, wherein the bung is configured to seal the decanting opening.
43. The milk collection device of clause 42, wherein the bung is movable between: a closed position wherein the bung is secured within the decanting opening; and an open position wherein the bung is not secured within the decanting opening, wherein in the open position the hinge is configured to bias the bung into a neutral position in which the bung is positioned out of the flow of the decanting milk path, and/or in which there is an angle of greater than 75 degrees between the plane of the decanting opening and the plane of the bung.
44. The milk collection device of any preceding clause, wherein the diaphragm is dome shaped.
45. The milk collection device of any preceding clause, wherein the breast shield is made from silicone.
46. An integrally formed diaphragm, hinge and bung for use with the device of any previous clause, wherein the diaphragm is attached to the bung via the hinge, wherein the bung is configured to seal an opening in the device.
47. A breast pump system comprising one or more milk collection devices according to any preceding clause connected to an air pump via the air line.
48. An in-bra wearable milk collection device for use in a breast pump system, the milk collection device comprising:
   a breast shield comprising a nipple tunnel and breast flange;
   a shell;
   a diaphragm comprising a dry side for connection to an air pump and a wet side configured to contact milk;
   wherein the breast shield is removably attachable to the circumference of the shell and when attached, the breast shield and shell form a milk container.
49. An in-bra wearable milk collection device for use in a breast pump system, the milk collection device comprising:
   a breast shield comprising a nipple tunnel and breast flange;
   a shell;
   a diaphragm comprising a dry side for connection to an air pump and a wet side configured to contact milk;
   wherein the breast shield is removably attachable to the shell and when attached, the breast shield and shell form a milk container;
   wherein the breast shield is made from a deformable material such that the breast shield is configured to deform to attach to the shell.
50. The milk collection device of clause 48 or 49, wherein the diaphragm is configured to attach to the shell such that the diaphragm seals the end of the nipple tunnel of the breast shield.
51. The milk collection device of clause 48, 49 or 50, wherein the breast shield is attachable to the shell in any orientation of the breast shield.
52. The milk collection device of any of clauses 48 to 51, wherein the circumference of the breast shield and circumference of the shell are different shapes.
53. The milk collection device of clause 52, wherein the circumference of the breast shield has an infinite order of rotational symmetry, and the circumference of the shell has a discrete order of rotational symmetry;
   and/or wherein the circumference of the breast shield is circular and the circumference of the shell is an oval shape.
54. The milk collection device of any one of clauses 48 to 53, comprising a rigid supporting member configured to support the nipple tunnel when the breast shield and shell are attached and/or when the device is in use.
55. The milk collection device of clause 54 wherein the shell comprises the rigid supporting member.
56. The milk collection device of clause 54 or 55, wherein the rigid supporting member is configured to support the user's breast and/or nipple when the device is in use.
57. The milk collection device of any of clauses 54 to 56, wherein the rigid supporting member comprises a cylindrical annulus.
58. The milk collection device of any of clauses 54 to 57 wherein the rigid supporting member comprises a conical region.
59. The milk collection device of any of clauses 48 to 58, wherein the material of the breast shield is thicker at the nipple tunnel than at the breast flange.
60. The milk collection device of any of clauses 48 to 59, wherein the breast flange comprises an inner annular portion and an outer annular portion, wherein the surface smoothness of the inner annular portion and the outer annular portion are different.
61. The milk collection device of clause 60, wherein the inner annular portion has a higher surface smoothness compared to the outer annular portion.
62. The milk collection device of any of clauses 48 to 61, wherein the diaphragm seals the end of the nipple tunnel via a seal circumferential to the shell and diaphragm, wherein the seal is configured such that in order for milk to overcome the seal, milk must flow in two opposite parallel directions.
63. The milk collection device of any of clauses 48 to 62, wherein when the milk collection device is assembled for use, the diaphragm is co-axial with the nipple tunnel.
64. The milk collection device of any of clauses 48 to 63, wherein when the milk collection device is assembled for use, the diaphragm is perpendicular to the nipple tunnel.
65. The milk collection device of any of clauses 48 to 64, wherein the device comprises a cap removably attachable to the diaphragm, wherein the cap is connectable to an air pump, and
   wherein an opening is formed between the cap and the shell on the lower half of the milk collection device when the device is orientated for use, such that milk which overcomes the seal will exit the device via the opening following gravity.
66. The milk collection device of any of clauses 48 to 65, wherein the only milk paths from the milk container to the dry side of the diaphragm are via an external side of the shell.
67. The milk collection device of any of clauses 48 to 66, wherein the shell comprises a decanting opening configured to pour milk from the milk container; and the diaphragm is attached to a bung via a hinge, wherein the bung is configured to seal the decanting opening.
68. The milk collection device of clause 67, wherein the diaphragm, hinge and bung form an integral component.
69. The milk collection device of clause 67 or 68, wherein the bung is movable between:
   a closed position wherein the bung is secured within the decanting opening; and
   an open position wherein the bung is not secured within the decanting opening,
   wherein in the open position the hinge is configured to bias the bung into a neutral position in which the bung is at an angle to the plane of the decanting opening of 75 degrees or greater.
70. The milk collection device of any of clauses 48 to 69, wherein the diaphragm is dome shaped.
71. A breast pump system comprising one or more milk collection devices according to any of clauses 48 to 70 connected to an air pump.

## Claims

1. An in-bra wearable milk collection device for use in a breast pump system, the milk collection device comprising:
a breast shield comprising a nipple tunnel and a breast flange;
a shell;
a diaphragm comprising a dry side for connection to an air pump and a wet side configured to contact milk;
wherein the breast shield is removably attachable to the shell and when attached the breast shield and shell form a milk container;
wherein the breast shield is made from a deformable material such that the breast shield is configured to deform to attach to the shell.

2. The milk collection device of claim 1, wherein the diaphragm is configured to attach to the shell such that the diaphragm seals the end of the nipple tunnel of the breast shield.

3. The milk collection device of any preceding claim, wherein the breast shield is attachable to the shell in any orientation of the breast shield.

4. The milk collection device of any preceding claim, wherein the circumference of the breast shield and circumference of the shell are different shapes.

5. The milk collection device of any preceding claim, comprising a rigid supporting member configured to support the nipple tunnel when the breast shield and shell are attached and/or when the device is in use.

6. The milk collection device of claim 5, wherein the shell comprises the rigid supporting member.

7. The milk collection device of claim 5 or 6, wherein the rigid supporting member is configured to support the user's breast and/or nipple when the device is in use.

8. The milk collection device of any of claims 5 to 7, wherein the rigid supporting member comprises a cylindrical annulus.

9. The milk collection device of any of claims 5 to 8, wherein the rigid supporting member comprises a conical region.

10. The milk collection device of any preceding claim, wherein the material of the breast shield is thicker at the nipple tunnel than at the breast flange.

11. The milk collection device of any preceding claim, wherein the breast flange comprises an inner annular portion and an outer annular portion, wherein the surface smoothness of the inner annular portion and the outer annular portion are different.

12. The milk collection device of any preceding claim, wherein the shell comprises a decanting opening configured to allow milk to be poured from the milk container via the decanting opening; and the diaphragm is attached to a bung via a hinge, wherein the bung is configured to seal the decanting opening.

13. The milk collection device of claim 12, wherein the diaphragm, hinge and bung form an integral component.

14. The milk collection device of claim 12 or 13, wherein the bung is movable between:
a closed position wherein the bung is secured within the decanting opening; and,
an open position wherein the bung is not secured within the decanting opening;
wherein in the open position the hinge is configured to bias the bung into a neutral position in which the bung is at an angle to the plane of the decanting opening of 75 degrees or greater.

15. A breast pump system comprising one or more milk collection devices according to any of claims 1 to 14 connected to an air pump.
